Europäisches Patentamt

European Patent Office

Office européen des brevets

(19)

(11) Veröffentlichungsnummer: **0 216 883**
**B1**

(12) # EUROPÄISCHE PATENTSCHRIFT

(45) Veröffentlichungstag der Patentschrift:
18.10.89

(51) Int. Cl.⁴: **A 61 F 5/34**

(21) Anmeldenummer: 86902381.2

(22) Anmeldetag: 24.03.86

(86) Internationale Anmeldenummer:
PCT/EP 86/00176

(87) Internationale Veröffentlichungsnummer:
WO 86/05385 (25.09.86 Gazette 86/21)

(54) **DRUCKVERBAND (ZUM STILLEN EINER BLUTENDEN ÄUSSEREN WUNDE) VENTIL.**

(30) Priorität: 23.03.85 DE 3510667

(43) Veröffentlichungstag der Anmeldung:
08.04.87 Patentblatt 87/15

(45) Bekanntmachung des Hinweises auf die Patenterteilung:
18.10.89 Patentblatt 89/42

(84) Benannte Vertragsstaaten:
AT BE CH DE FR GB IT LI LU NL SE

(56) Entgegenhaltungen:
DE-A- 2 948 059
US-A- 2 674 064
US-A- 2 949 927
US-A- 3 171 410
US-A- 4 393 867

(73) Patentinhaber: MAASS, Walter, Bernd, Hohenlohestr. 26,
D-2800 Bremen (DE)

(72) Erfinder: MAASS, Walter, Bernd, Hohenlohestr. 26,
D-2800 Bremen (DE)

(74) Vertreter: Hoormann, Walter, Dr.-Ing. et al, FORRESTER
& BOEHMERT Widenmayerstrasse 4/I,
D-8000 München 22 (DE)

## Beschreibung

Die Erfindung betrifft einen Druckverband zum Stillen einer blutenden äußeren Wunde, mit einer hohlen, gasdichten Kompresse, die wenigstens einen mit seiner Außenseite auf die zu versorgende Wunde aufzulegenden, elastisch verformbaren Wandungsabschnitt aufweist, der durch innenseitige Beaufschlagung mit einem unter Überdruck stehenden Strömungsmittel nach außen zu Verformen ist, wobei die Kompresse zur Erzeugung des inneren Überdrucks mit einem Ventil versehen ist (s. US-A-3 171 410).

Wenn vor- oder nachstehend von einem «Druckverband» die Rede ist, so soll hierunter nicht ausschließlich ein selbst haltender Verband gemeint sein, dessen Kompresse durch eine Mullbinde, ein Heftpflaster oder dgl. am Körper eines Patienten gehalten ist. Vielmehr kann der erfindungsgemäße Druckverband, wie für den einschlägigen Fachmann ohne weiteres hervorgehen wird, bei speziellen Anwendungsfällen auch ohne besondere Befestigungsmittel verwandt werden. Ein solcher Anwendungsfall kann beispielsweise bei der Versorgung eines Zahnwurzelkanals nach Extraktion eines Zahnes vorliegen.

Wenn vor- oder nachstehend von einer «Kompresse» die Rede ist, so ist hiermit ein Element gemeint, mit dem auf eine zu versorgende Wunde eine gewisse Kompression auszuüben ist, um eine Blutung zu verhindern oder eine bereits eingetretene Blutung zu stillen.

Sogenannte Kompressions- oder Druckverbände sind seit eh und je zur Versorgung von Wunden bekannt. Hierzu wird lediglich beispielsweise auf die DE-PS 188 337 oder die DE-PS 550 751 verwiesen. Bei diesen seit vielen Jahrzehnten bekannten (Druck-)«Verbänden» besteht die Kompresse aus textilem oder textilähnlichem Material, beispielsweise aus mit Gaze umhüllter Watte, Mull oder dgl.

Hieran hat sich über viele Jahre grundsätzlich nichts geändert. So schreibt auch beispielsweise die auf «Brandwunden-Verbandpäckchen» gerichtete DIN 13 153 aus dem Jahre 1965 vor, daß diese aus einem Zellwollgewebe in Leinwandbindung bestimmter Ausgestaltung besteht. Entsprechendes gilt beispielsweise für die Verbandkompressen der Bundeswehr (s. «Technische Lieferbedingungen» TL 6510-002/3 des Bundesamtes für Wehrtechnik und Beschaffung aus dem Jahre 1967).

Auch neuere Entwicklungen für Druckverbände (bzw. zumindest alternativ hierfür bestimmte und geeignete Verbände) weisen als Kompresse einen blutstillenden Druckkörper auf, der aus einer aufgewickelten Mullbinde o.ä. besteht (s. z.B. DE-AS 2 840 667, angemeldet im Jahre 1978 und veröffentlicht im Jahre 1981).

Obwohl die Kompressen vorbekannter und bis zum heutigen Tage in millionenfacher Anwendung verwandter Druckverbände sich in einer Vielzahl von Fällen bestens bewährt haben, verbleibt eine unbeachtliche Anzahl von Anwendungsfällen, in denen konventionelle Druckverbände versagen bzw. zu teilweise ganz erheblichen Schwierigkeiten führen. Solche Fälle kommen im professionelle Bereich, wie z.B. in der Dentalversorgung, tagtäglich vor, wenn Patienten beispielsweise ein Zahn extrahiert worden ist, ohne daß es sich bei derartigen Patienten um solche mit ungewöhnlich niedriger Blutgerinnungsfähigkeit ( = Bluter oder Quasi-Bluter) handeln muß, aber auch in Erste-Hilfe-Fällen, in denen Wunden in aller Regel zunächst durch Laien provisorisch zu versorgen sind.

Bei einem derartigen Nachbluten von (insbesonderen äußeren) Wunden bringt der behandelnde Arzt bisher eine aus Gaze oder dgl. bestehende sog. Drucktamponate auf und veranlaßt den betreffenden Patienten beispielsweise nach Extraktion eines Zahnes, Ober- und Unterkiefer über ein bestimmtes (im allgemeinen längeres) Zeitintervall fest aneinander zu pressen, um die Blutung zum Stillstand zu bringen. Abgesehen davon, daß dieses bei zahlreichen Patienten im Zusammenhang mit einem gewissen psychischen Streß zu krampfartigen Zuständen im Kieferbereich führen kann, führt diese Maßnahme auch häufig genug nicht einmal zu dem gewünschten Ziel, nämlich einer alsbaldigen Blutstillung der betreffenden Wunde.

Entsprechendes gilt für eine Vielzahl anderer medizinischer Anwendungsfälle, von denen lediglich beispielhaft die bisherige Versorgung des Brustkastens nach vorheriger Eröffnung mittels eines sog. Dachziegelverbandes (empfohlen bzw. vorgeschrieben vom DRK) oder die Versorgung einer geöffneten Halsschlagader genannt werden sollen, wobei der letztgenannte Fall bekanntlich nicht nur wegen des verhältnismäßig hohen Blutverlustes besonders kritisch ist, sondern auch deswegen, weil aufgrund des Unterdruckes in der Halsschlagader die große Gefahr eines Ansaugens von Luft aus der Umgebung mit den bekannten Folgen besteht.

Zur Vermeidung der mit konventionellen Druckverbänden der vorstehend beschriebenen Art auftretenden Schwierigkeiten ist aus der DE-OS 3 145 110 eine anatomische Druckbandage zum Stabilisieren von Knochenbrüchen sowie zum Kontrollieren einer Wundblutung bekanntgeworden, die eine flexible, expandierbare Hülle aufweist, welche wenigstens zwei chemische Reagenzien enthält, die bei einer Vermischung ein Gas bilden, mit dem im Anwendungsfalle in der im wesentlichen aus dieser Hülle bestehenden Kompresse ein Überdruck erzeugt werden soll. Bei der Anwendung umgibt die Hülle ein Körperteil (beispielsweise ein verwundetes Bein oder einen verwundeten Arm), wobei die chemischen Reagenzien durch eine manuelle Beeinflussung der Hülle zu einer Vermischung gebracht werden sollen, um auf diese Weise ein Gas zu erzeugen, welches die Hülle aufbläst, um auf das zu versorgende Körperteil einen Druck auszuüben.

Diese bekannte Druckbandage ist u.a. schon deswegen nachteilig, weil sich eine der im Anwendungsfalle zur Reaktion zu bringenden Reagenzien in Kapseln befindet, die im Anwendungsfalle durch Einwirkung von außen her zerbrochen werden sollen. Dabei kommt es bekanntlich zwangsläufig zu scharfen Kanten, die zu einer Perforierung der Kompresse führen können, so daß diese nicht mehr gebrauchsfähig ist.

Es kommt hinzu, daß in einem solchen Falle zwangsläufig das mittels der chemischen Reagenzien entstandene Gas aus der Kompresse austritt und auf die zu versorgende Wunde gelangen kann, was in zahlreichen Fällen außerordentlich nachteilig sein kann.

Selbst wenn es jedoch im Anwendungsfalle bei dieser bekannten Druckbandage nicht zu einer Beschädigung der Kompresse kommt, so ist sie dennoch extrem nachteilig, weil die vorgesehene Steuerung des Kompresseninnendruckes höchst mangelhaft ist. Stellt man nämlich nach dem Zerbrechen einer Kapsel fest, daß der hiermit erzeugte Innendruck in der Kompresse nicht groß genug ist, und will — wie dieses bei der bekannten Kompresse für einen solchen Fall vorgesehen ist — eine zweite Kapsel zerbrechen, um durch chemische Reaktion mehr Gas, und damit einen höheren Kompresseninnendruck zu erzeugen, so läßt sich eine weitere Kapsel im teilweise aufgeblasenen Zustand der Kompresse überhaupt nicht auffinden. Selbst wenn man sie aber auffindet, kann es bei bereits gedehnter Kompresse besonders leicht zu einer Beschädigung und zu einem Unbrauchbarwerden kommen. Selbst wenn dieses nicht geschehen sollte, ist der optimal einzustellende Druck nur äußerst schlecht steuerbar, da ein Zerbrechen einer zweiten oder gar dritten Kapsel, sofern man diese überhaupt auffindet und im angelegten Zustand zerbrechen kann, den Innendruck nicht kontinuierlich bis zum Optimalpunkt erhöht, sondern stufenweise, so daß es sehr schnell zu einem zu hohen Druck kommen kann. Zwar ist in Ausgestaltung dieser bekannten Druckbandage vorgesehen, daß in einem solchen Falle ein Ablaßventil betätigt werden kann, doch kommt es dabei dann sehr schnell — insbesondere, wenn eine solche Druckbandage in einem Erste-Hilfe-Fall von Laien oder gar dem Patienten selbst in einer Streßsituation angelegt wird — zu einem übermäßigen Ablassen von chemisch erzeugtem Gas und somit wiederum zu einem zu schlaffen Zustand der Kompresse, der dann aber nicht mehr veränderbar ist, wenn die in der Kompresse vorhandenen Kapseln aufgebraucht sind.

Weiterhin kommt bei dieser vorbekannten Druckbandage als nachteilig hinzu, daß sie im Bevorratungszustand höchst schonend verwahrt werden und keinem Druck ausgesetzt werden darf, da es in einem solchen Falle — insbesondere nach längerer Aufbewahrungszeit — zu einem unbeabsichtigten Zerbrechen von Kapseln, und damit zu einem Aufblasen der Kompresse beispielsweise in einem Verbandspäckchen oder -kasten kommen kann, was die bekannte Druckbandage nicht nur unbrauchbar macht, sondern auch höchst nachteilige Nebenwirkungen hervorrufen kann, wenn es sich bei dem Verbandskasten beispielsweise um einen in einem Kraftfahrzeug mitgeführten Verbandskasten oder bei dem Verbandspäckchen beispielsweise um ein von einem Soldaten mitgeführtes Verbandszeug handelt.

Aber selbst wenn man einmal unterstellt, daß es hierzu nicht käme, und daß die im Inneren der Kompresse befindlichen chemischen Reagenzien während der Aufbewahrungszeit nicht unwirksam werden, ist weiterhin noch als nachteilig festzustellen, daß diese bekannte Druckbandage im Bevorratungszustand einen relativ großen Platzbedarf erfordert, daß sie druckgeschützt aufbewahrt werden muß etc.

Der vorliegenden Erfindung liegt demgemäß die Aufgabe zugrunde, den bekannten Druckverban der eingangs beschriebenen Gattung unter Vermeidung seiner vorgenannten und weiterer Nachteile insbesondere dahingehend zu verbessern, daß er sowohl bei professioneller medizinischer Versorgung als auch im Erste-Hilfe-Fall von Laien in sicherer, einfacher und für die Wundbehandlung optimaler Weise unter entsprechender Steuerung des den Druck auf die zu versorgende Wunde bewirkenden Innendruckes der Kompresse zu verwenden ist, wobei ein ggf. zunächst zu geringer Kompresseninnendruck ohne Schwierigkeit stetig zu erhöhen und ein ggf. zu großer Kompressendruck auf einfache Art und Weise stetig zu vermindern sein soll, um den auf die zu versorgende Wunde auszuübenden Druck letztlich im angelegten Zustand nur so groß bzw. lediglich etwas größer machen zu können als es dem Druck der verletzten Blutgefäße entspricht, wobei darüber hinaus der erfindungsgemäße Druckverband insbesondere im Hinblick auf seine für Erste-Hilfe-Anwendungen erforderliche Massenherstellung trotz seiner Vorzüge möglichst billig herzustellen und im Bevorratungszustand äußerst raumsparend unterzubringen sein soll, und wobei sicherzustellen sein soll, daß durch den Druckverband keinerlei schädliche Wirkungen auf eine zu versorgende Wunde ausgeübt werden.

Als Lösung dieser Aufgabe ist erfindungsgemäß vorgesehen, daß der Ventilkörper des Ventils aus einer flexiblen Ventilscheibe besteht, die an der an den Strömungsquerschnitt angrenzenden Wandung einer Strömungsmittel-Zuführleitung für die Kompresse so befestigt ist, daß sie bei einem inneren Überdruck der Kompresse dichtend an die an den Strömungsquerschnitt angrenzende, einen Ventilsitz bildende Wandung anzudrücken ist, und daß sie bei einem äußeren Überdruck an wenigstens einem Abschnitt des Ventilsitzes außer Anlage mit dem Ventilsitz zu bringen ist. Die Wirkung dieser Lösung ist daß mithin unter Überdruck stehendes Strömungsmittel von außen in das Innere der Kompresse strömen kann oder beim Einstellen eines zunächst zu hohen Kompresseninnendruckes auf einen optimalen Druckwert auch umgekehrt, d.h. also von innen nach außen, wie dieses weiter unten noch im einzelnen erläutert ist.

Die flexible Ventilscheibe besteht bevorzugt aus einem gummi-elastischen Material, um eine gute Dichtigkeit zu erreichen, wobei dieser Effekt ersichtlich auch dann zu erreichen ist, wenn dieser Ventilsitz aus einem solchen Material besteht, wobei sich eine besonders gute Dichtwirkung bei einer Ausgestaltung herausgestellt hat, bei welcher sowohl die Ventilscheibe als auch der Ventilsitz gummi-elastisch ausgebildet sind, und zwar insbesondere dann, wenn der Ventilsitz gemäß einer bevorzugten Ausführung der vorliegenden Erfindung ringförmig ausgebildet ist. Dieses kann bei einer bestimmten Ausgestaltung der vorliegenden Erfindung der Fall sein, bei welcher die der Ventilscheibe zugekehrte Stirnseite eines Abschnittes der Strömungsmittel-Zuführleitung oder eines an dieser angeordneten Flansches den Ventilsitz bildet.

Eine bei größter Zuverlässigkeit extrem einfache und demgemäß auch entsprechend billig herzustellende sowie darüber hinaus praktisch verschleiß- und wartungsfreie Ausbildung besteht erfindungsgemäß darin, daß die Ventilscheibe an einem Umfangsabschnitt ihres äußeren Randabschnittes mit der Strömungsmittel-Zuführleitung gasdicht verbunden ist, was in Ausgestaltung der vorliegenden Erfindung auch an mehreren, jeweils mit Abstand zueinander angeordneten Umfangsabschnitten der Fall sein kann, wobei der bzw. ein nicht mit der Strömungsmittel-Zuführleitung verbundener Umfangsabschnitt der Ventilscheibe bevorzugt (erheblich) kleiner ist als der (die) mit der Strömungsmittel-Zuführleitung gasdicht verbundenen Umfangsabschnitt(e).

Um das Ventil des erfindungsgemäßen Druckverbandes nicht allein als Rückschlagventil wirken zu lassen, sondern auch als Steuerventil für den inneren Überdruck der Kompresse in vorteilhafter Weise benutzen zu können, sieht die Erfindung in bevorzugter Ausgestaltung weiterhin vor, daß die Ventilscheibe im Bereich eines nicht mit der Strömungsmittel-Zuführleitung verbundenen Randabschnittes mit einer Handhabe versehen ist, mittels welcher der nicht mit der Strömungsmittel-Zuführleitung verbundene Randabschnitt auch und insbesondere bei innerem Überdruck der Kompresse außer Anlage mit dem Ventilsitz zu bringen ist, wie weiter unten noch im einzelnen erläutert ist, so daß bei Betätigung der Handhabe von außen ein ggf. in der Kompresse herrschender zu hoher innerer Überdruck durch Betätigung der Handhabe zu reduzieren ist. Eine derartige Handhabe kann gemäß einer Ausführungsform der vorliegenden Erfindung integraler Bestandteil der Ventilscheibe sein, und zwar insbesondere bei einer solchen Ausbildung als ein nach außen über die Strömungsmittel-Zuführleitung vorstehender Ansatz ausgebildet sein, der sich bevorzugt durch eine Durchgangsöffnung in der Strömungsmittel-Zuführleitung erstreckt, die ihrerseits mittels einer gummielastischen Membran oder dgl. nach außen gasdicht abgedichtet sein kann, welche bevorzugt an ihrem Randumlauf mit der Außenseite der Strömungsmittel-Zuführleitung verbunden sein, kann.

Um insbesondere bei relativ großem Strömungsquerschnitt relativ dünner Ventilscheibe, bei der es sich — wenn sie beispielsweise aus einem äußerst dünnen Folienabschnitt besteht — nicht um eine «Scheibe» im klassischen Sinne zu handeln braucht, zu verhindern, daß die Ventilscheibe aufgrund der herrschenden Druckverhältnisse gleichsam durch den Ventilsitz hindurchgedrückt bzw. -gesaugt wird, kann in weiterer Ausgestaltung der vorliegenden Erfindung auf der der Ventilscheibe abgekehrten Seite des Ventilsitzes eine Ventilscheibenstütze vorgesehen sein, für welche sich unterschiedlichste Ausgestaltungen als sehr geeignet erwiesen haben.

Weitere bevorzugte Ausgestaltungen der vorliegenden Erfindung sind in den Unteransprüchen beschrieben.

Die Erfindung ist nachstehend an Ausführungsbeispielen unter Bezugnahme auf eine Zeichnung weiter erläutert. Es zeigt:

Fig. 1 eine schematische Prinzipdarstellung eines erfindungsgemäßen Druckverbandes mit einem elastisch verformbaren Wandungsabschnitt in einer Schnittdarstellung;

Fig. 2 eine schematische Darstellung des Druckverbandes gemäß Fig. 1 in einem Anwendungsfall;

Fig. 3 eine kissenförmige Ausgestaltung des erfindungsgemäßen Druckverbandes mit insgesamt elastisch verformbarer Kompresse;

Fig. 4 eine Variante des erfindungsgemäßen Druckverbandes in balgförmiger Ausbildung mit festen, aber gepolsterten Druckplatten;

Fig. 5 eine weitere Variante in schematischer Darstellung in Anwendung bei einer blutstillenden Versorgung einer Halsschlagaderverletzung;

Fig. 6 eine insbesondere für Erste-Hilfe-Zwecke bestimmte Variante des erfindungsgemäßen Druckverbandes in einer Seitenansicht gemäß dem Pfeil VI in Fig. 7;

Fig. 7 eine Draufsicht auf den Druckverband gemäß Fig. 6 in Richtung des Pfeiles VII in Fig. 6;

Fig. 8 eine vergrößerte Teildarstellung des in den Fig. 6 und 7 mit einem strichpunktierten Kreis umrahmten und mit VIII bezeichneten Abschnittes, also des in die Strömungsmittel-Zuführleitung eingebauten Ventils;

Fig. 9 eine Schnittdarstellung durch den Gegenstand von Fig. 8 in Richtung der Schnittlinie IX-IX in Fig. 8 gesehen;

Fig. 10 eine Schnittdarstellung durch den Gegenstand gemäß Fig. 8 in Richtung der Schnittlinie X-X in Fig. 8 gesehen; und

Fig. 11 eine Draufsicht auf den Gegenstand gemäß Fig. 8 in Richtung des Pfeiles XI in Fig. 8 gesehen.

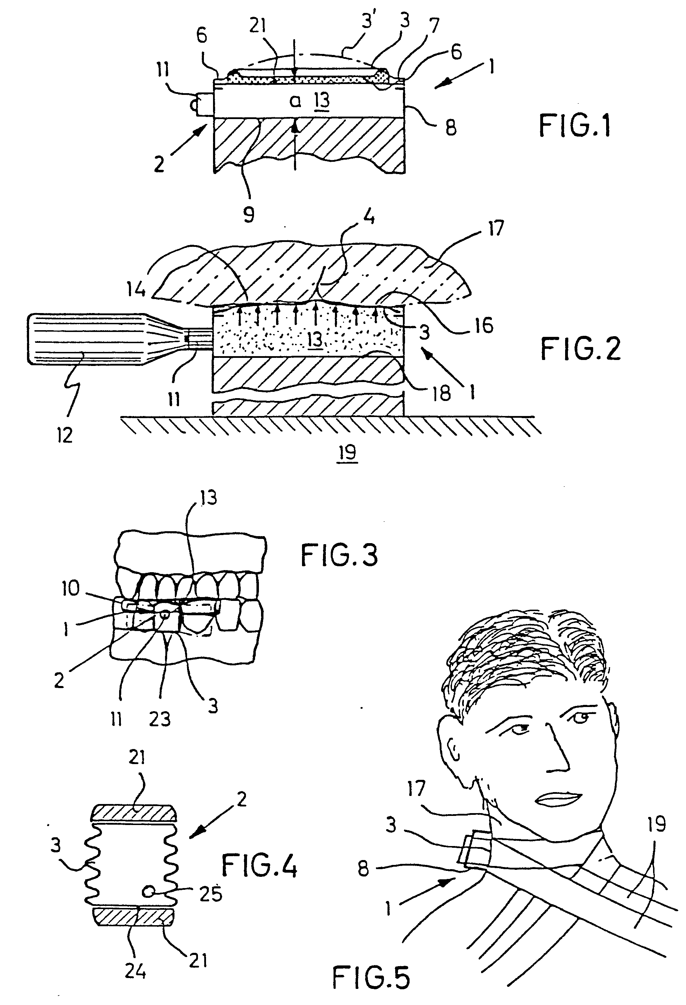

Fig. 1 der Zeichnung zeigt eine schematische Darstellung eines im ganzen mit 1 bezeichneten erfindungsgemäßen Druckverbandes zum Stillen einer Blutung einer äußeren Wunde. Der Druckverband 1 besteht im wesentlichen aus einer auf einer zu versorgenden Wunde unter Druckauflage anzuordnenden Kompresse 2. Die Kompresse 2 ist gasdicht ausgebildet. Sie weist einen Wandungsabschnitt 3 auf, der mit seiner Außenseite auf die zu versorgende Wunde 4 (s. Fig. 2) aufzulegen ist und durch innenseitige Beaufschlagung mit einem unter Überdruck stehenden Strömungsmittel nach außen zu verformen ist.

Bei dem Ausführungsbeispiel gemäß den Fig. 1 und 2 besteht der unter innerem Überdruck elastisch verformbare Wandungsabschnitt 3 aus einer Gummimischung und ist aus einem bahnförmigen Material geschnitten, wobei er in der Art einer Membran ausgebildet ist, die mit ihrem Rand 6 fest — und zwar gasdicht — in Flansche 7 eines Rahmens 8 eingespannt ist. Zwischen dem elastisch verformbaren Wandungsabschnitt 3 und der mit Abstand a zu diesem angeordneten, gegenüberliegenden Wandung 9 der Kompresse 2 ist ein Ventil 11 angeordnet, welches (s. Fig. 2) an eine (kleine) handelsübliche $CO_2$-Flasche 12 anzuschließen ist, die als (Gas-)Druckmittelreservoir dient, mittels dessen in der Kammer 13 zwischen dem elastischen Wandungsabschnitt 3 und der gegenüberliegenden Wandung 9 ein Überdruck gegenüber dem Umgebungsdruck (= Atmosphärendruck) zu erzeugen ist. Wird die Gasflasche 12 an das Ventil 11 angeschlossen, so wölbt sich der elastische Wandungsabschnitt 3 in der in Fig. 1 mit einer strichpunktierten Linie 3' bezeichneten Weise nach außen aus.

Fig. 2 zeigt den Druckverband 1 (mit angeschlossener Gasflasche 12) schematisch in Anwendung. Dabei liegt mithin die Außenseite 14 des elastischen Wandungsabschnittes 3 der Kompresse 2 an dem die betreffende Wunde 4 enthaltenden Hautabschnitt 16 des betreffenden Patienten 17 an, während die gegenüberliegende Außenseite 18 der Kompresse von einem «Widerlager» 19 abgestützt wird. Bei diesem «Widerlager» 19 kann es sich beispielsweise um einen klassischen (Mull-)Verband, ein (größeres) Heftpflaster oder aber auch um ein Körperteil des betreffenden Patienten (beispielsweise auch um dessen Hand) handeln.

Sobald die Außenseite 14 des elastischen Wandungsabschnittes 3 an dem Hautabschnitt 16 des Patienten 17, und damit an der Wunde 4, anliegt, wird die Gasflasche 12 an das Rückschlagventil 11 angeschlossen, so daß es in der Kammer 13 zum Aufbau eines Überdruckes und damit zu einer Druckausübung des elastischen Wandungsabschnittes 3 auf den Hautabschnitt 16 und damit die Wunde 4 kommt.

Es sei noch darauf verwiesen, daß die der Wunde zugekehrte Außenseite 14 des elastischen Wandungsabschnittes 3 mit einer Polsterung 21 aus Gaze und ggf. Watte versehen sein kann, um eine Aufnahmekapazität für aufzusaugendes Blut zu schaffen, wie dieses von Wundverbänden her an sich grundsätzlich bekannt ist.

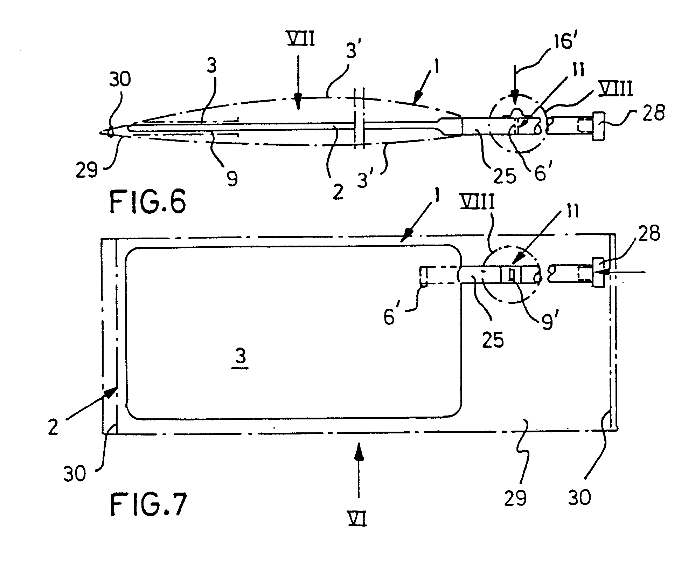

Fig. 3 zeigt eine Variante eines erfindungsgemäßen Druckverbandes 1 in schematischer Darstellung. Bei dieser Variante besitzt die Kompresse 2 an mehreren nicht in einer Ebene liegenden Oberflächenabschnitten Wandungsabschnitte, die unter Strömungsmittel-Innendruck nach außen verformbar sind, und zwar sowohl Wandungsabschnitte, die parallel zueinander verlaufen, als auch verformbare Oberflächenabschnitte, die im Winkel zueinander verlaufen. Denn die Kompresse 2 gemäß Fig. 3 ist im wesentlichen an ihrer gesamten Oberfläche unter Strömungsmittel-Innendruck nach außen verformbar, d.h. gleichsam kissenförmig ausgebildet, und zwar in ihrem mittleren Abschnitt annähernd würfelförmig, wobei sich die an den mittleren Abschnitt anschließenden, in Fig. 3 strichpunktiert dargestellten Endabschnitte verbreitert sind, so daß die Kompresse 2 formschlüssig in einer Zahnlücke gehalten ist. Als Widerlager 19 dient die Kappe 10, die von oben her auf die Kompresse 2 aufzusetzen ist und als Abschnitt eines Abdrucklöffels ausgebildet sein kann. Die Kompresse 2 besteht aus einem gummiartigen Folienmaterial, welches an seiner einen Seitenfläche mit einem Ventil 11 versehen ist, jedoch auch in einer Strömungsmittel-Zuführleitung angeordnet sein könnte, wie dieses regelmäßig der Fall ist (s. z.B. Leitung 25 in den Fig. 6 bis 11).

Der Druckverband 1 gemäß Fig. 3 dient zur Versorgung einer nach erfolgter Zahnextraktion entstandenen Wunde eines Zahnwurzelkanals zwecks Blutstillung. Die Kompresse 2 wird unmittelbar nach erfolgter Extraktion und Säuberung der Wunde in die Zahnlücke eingebracht und mit einer Kappe 10 überzogen. Sodann wird der betreffende Patient veranlaßt, Ober- und Unterkiefer leicht aufeinanderzudrücken, um die Kappe 10 in Stellung zu halten. Danach wird Gas aus einer an das Rückschlagventil 11 anzuschließenden Gasflasche 12 (nicht dargestellt) in die Kammer 13 der Kompresse 2 eingelassen, so daß sich oben an der Kappe 10 und an den Seitenflächen der benachbart zur Zahnlücke 23 befindlichen Zähne abstützt. Der der Zahnlücke 23 zugekehrte elastische Wandabschnitt 3 drückt dabei unter dem Einfluß des in die Kammer 13 eingelassenen, unter Überdruck stehenden Gases dichtend auf die Wunde und hat eine überraschend blutstillende Wirkung, so daß es in aller Regel bereits nach kurzer Zeit zu einer Beendigung einer Nachblutung kommt.

Fig. 4 zeigt ebenfalls in schematischer Darstellung eine Variante, bei welcher der verformbare Wandungsabschnitt 3 balgartig ausgebildet ist, wobei im Falle einer solchen Ausbildung nicht der elastische Wandabschnitt 3 unmittelbar auf die zu versorgende Wunde wirkt, sondern im Zusammenwirken mit dem Strömungsmittel-Innendruck gleichsam als Feder wirkt, während die kopf- und fußseitigen Stirnplatten 24 fest ausgebildet und jeweils mit einer Polsterung 21 versehen sind, wobei eine Polsterung 21 unter dem entstehenden Druck blutstillend auf die zu versorgende Wunde wirkt.

Fig. 5 zeigt eine weitere Variante eines erfindungsgemäßen Druckverbandes 1 mit einem Rahmen 8, in den an der Unterseite (und ggf. an der Oberseite) ein elastischer Wandungsabschnitt 3 in der Art einer Membran gasdicht eingespannt ist. Das «Widerlager» 19 für den Druckverband 1 bzw. die Kompresse besteht im vorliegenden — schematisch dargestellten — Fall aus einer Mullbinde, mittels welcher der Druckverband 1 am Hals des betreffenden Patienten 17 befestigt ist, um dessen verletzte offene Schlagader abzudichten und dabei außer vor einem übermäßigen Blutverlust gegen ein Eindringen von Luft in die Halsschlagader zu sichern.

Bei dem insbesondere für Erste-Hilfe-Zwecke bestimmten und geeigneten Ausführungsbeispiel gemäß den Fig. 6 und 7 besteht die Kompresse 2 insgesamt aus einer Gummimischung und ist aus bahnförmigem Material geschnitten, so daß die Wandung der hohlkissenförmigen Kompresse 2 jeweils in der Art einer Membran ausgebildet ist. Zwischen dem oberen Wandungsabschnitt 3 und dem darunterliegenden Wandungsabschnitt 9 der Kompresse 2 mündet eine ein Ventil 11 enthaltende Strömungsmittel-Zuführleitung 25 an einer Stirnseite seitlich in die Kompresse 2 ein. Die Strömungsmittel-Zuführleitung 25 besteht aus einem Gummischlauch und ist an ihrem freien Endabschnitt entweder mit dem Mund oder aber einer kleinen Pumpe bzw. ggf., falls erwünscht, auch mit einer kleinen Gasflasche im angelegten Zustand aufzublasen, nachdem die Kompresse 2 mit ihren Wandungsabschnitt 3 oder 9 auf eine zu versorgende Wunde aufgelegt und dort beispielsweise mit einer Mullbinde fixiert worden ist. Dabei entsteht mithin in der Kammer zwischen den elastischen Wandungsabschnitten 3 und 9 ein Überdruck gegenüber dem Umgebungsdruck ( = Atmosphärendruck), so daß sich die elastischen Wandungsabschnitte 3, 9, sofern sie hieran nicht durch die Mullbinde gehindert sind, in der in Fig. 6 mit einer strichpunktierten Linie 3' angedeuteten Weise nach außen wölben. In Fig. 7 ist mit strichpunktierten Linien eine beutelförmige Umhüllung 29 für den Druckverband 1 angeordnet, die mit einer Schweißnaht 30 verschlossen ist.

Es sei noch darauf verwiesen, daß auch bei einer derartigen Ausgestaltung die der Wunde zugekehrte Außenseite des betreffenden elastischen Wandungsabschnittes 3 bzw. 9 mit einer Polsterung aus Gaze oder/und Watte versehen sein kann, wie dieses bei der Ausgestaltung gemäß Fig. 4 bezüglich der Polsterung 21 der Fall ist, und zwar u.a. um eine Aufnahmekapazität für aufzusaugendes Blut zu schaffen, wie dieses von Wundverbänden her an sich grundsätzlich bekannt ist.

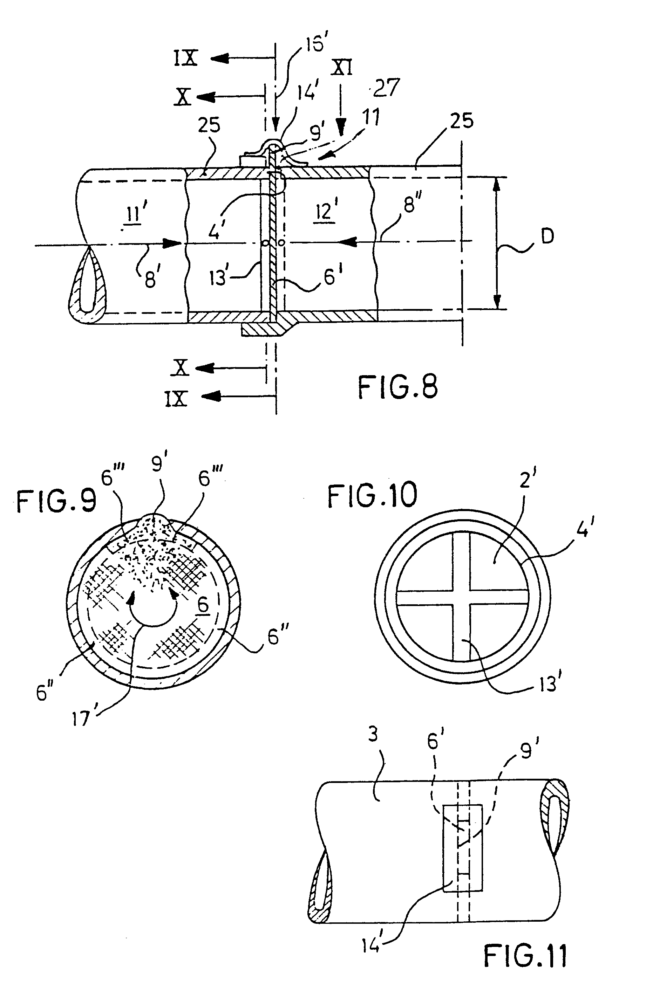

Die Fig. 8 bis 10 zeigen das im ganzen mit 11 bezeichnete Ventil, mittels dessen der kreisförmige Strömungsquerschnitt 2' der Leitung 25 (mit dem Durchmesser D) entweder abzusperren oder freizugeben ist. Der Strömungsquerschnitt 2' ist von der Leitung 25 begrenzt, deren eine Stirnseite einen ringförmigen Ventilsitz 4' bildet, der ggf. mit einem gummielastischen Material belegt sein kann.

Dem Ventilsitz 4' ist eine als Ventilkörper wirkende Ventilscheibe 6' zugeordnet, die aus einer relativ dünnen, flexiblen «Membran» aus einem gummielastischen Material besteht. Die Ventilscheibe 6' ist über einen Zentriwinkel 17' von etwa 300° mit einem Ventilsitz 4', also der Stirnseite der Leitung 25, fest verklebt, so daß mithin lediglich ein Abschnitt 6''' von etwa 60° lose auf bzw. an dem Ventilsitz 4' auf- bzw. anliegt.

In einer ersten Betriebsstellung ist das Ventil 11 ein bei einem in Richtung des Pfeiles 8' auf den Ventilsitz 4' gerichteten Strömungsmitteldruck geschlossen, da die Ventilscheibe 6' mit ihrem nicht mit der Stirnseite (= Ventilsitz 4') der Leitung 25 fest (gasdicht) verbundenen Randabschnitt an den Ventilsitz 4' angedrückt wird, so daß eine Strömungsmittelströmung in dieser ersten Strömungsrichtung 8' verhindert wird.

Bei umgekehrtem Überdruck in Richtung des Pfeiles 8'' bewirkt dieser, daß sich der nicht fest mit dem Ventilsitz 4' verbundene Randabschnitt der Ventilscheibe 6' (der mit einer integral an die Ventilscheibe 6' angeformten Handhabe 9' versehen ist) vom Ventilsitz 4' abhebt und demgemäß eine entsprechende Strömungsmittelströmung in der zweiten Strömungsrichtung 8'' ermöglicht, wie dieses beim Aufblasen erforderlich ist. Über diese Rückschlagwirkung hinaus ist das Ventil 11 aber auch in der erstgenannten Betriebsstellung zu öffnen, wenn der Überdruck in der Kompresse 2 vermindert werden soll. Hierfür drückt man auf die Handhabe 9', wodurch sich die Ventilscheibe 6' vom Ventilsitz 4' abhebt, also das Ventil 11 öffnet.

Wie aus der Zeichnung erkennbar ist, ist die Ventilfläche des Ventilsitzes 4' kreisringförmig, während die Ventilscheibe 6' in der Art einer flachen, elastischen, flexiblen Scheibe 6' ausgestaltet ist.

Um zu verhindern, daß die Handhabe 9' bei einem zu großen (Unter- oder Über-)Druck entweder in die in Fig. 8 links von der Ventilscheibe 6' liegende Kammer 11' oder in die in Fig. 6 rechts von der Ventilscheibe 6' liegende Kammer 12' hineingedrückt bzw. hineingesaugt wird, ist eine Ventilscheibenstütze 13' vorgesehen, die in der Art eines «Fadenkreuzes» ausgebildet ist (s. Fig. 10), die jedoch auch beispielsweise Y-förmig oder auf andere Art und Weise gasdurchlässig und gleichzeitig stützend für die Ventilscheibe 6' wirkend ausgebildet sein kann.

An der Außenseite der elastischen Strömungsmittelleitung 25 befindet sich an der muffeartigen Verbindungsstelle ein schlitzförmiger Durchlaß 27, durch welchen sich die integral mit der Ventilscheibe 6' ausgebildete Handhabe 9' hindurcherstreckt, so daß sie mit einem relativ kleinen Abschnitt über die Leitung 25 nach außen vorsteht. Der freie Endabschnitt der Handhabe 9' ist mit einem Folienabschnitt 14' gasdicht verklebt.

Das erfindungsgemäße Ventil erfüllt mithin trotz einfachen Aufbaus, Wartungsfreiheit, einer geringen Anzahl relativ zueinander beweglicher Teile, einer flexiblen Ausgestaltung etc. die an ein derartiges Ventil zu stellenden Anforderungen und ist trotz dieser Lösungsmöglichkeit vielfältiger Aufgaben relativ preiswert zu erstellen. Es bewährt sich bei dem erfindungsgemäßen Druckverband schließlich auch deshalb besonders, weil es das Kompressenmaterial nicht beschädigen kann (insbesondere, wenn es in einem flexiblen Schlauch eingebaut ist), nicht korrodiert, nicht brechen kann etc. und weil es die erstrebte extrem raumsparende Vorratshaltung in keiner Weise nachteilig beeinträchtigt.

Es sei noch darauf hingewiesen, daß der erfindungsgemäße Druckverband 1 unter Verwendung des erfindungsgemäßen Ventils 11 seinen Kompressenüberdruck über einen erstaunlich langen Zeitraum aufrechterhält. Um ggf. gewisse Leckverluste (also einen unbeabsichtigten Druckausgleich) noch weiter hinauszuzögern, kann in den freien Endabschnitt einer Strömungsmittel-Zuführleitung 25 ggf. ein stöpselförmiger Verschlußkörper 28 eingesetzt werden, der bei einem ggf. erforderlich werdenden Nach-Aufblasen in einfachster Weise zu entfernen und ggf. wieder in die Strömungsmittel-Zuführleitung 25 einzustöpseln ist.

*Bezugszeichenliste*
(List of Reference Numerals)

| | |
|---|---|
| 1 | Druckverband |
| 2 | Kompresse |
| 2' | (freier) Strömungsquerschnitt (von 25) |
| 3 | Wandungsabschnitt (von 2) |
| 4 | Wunde |
| 4' | Ventilsitz |
| 5 | — |
| 6 | Rand (von 3) |
| 6' | Ventilscheibe (von 11) |
| 6'' | Randabschnitt (von 6'; mit 4' verbunden) |
| 6''' | Randabschnitt (von 6'; nicht mit 4' verbunden) |
| 7 | Flansche (von 8) |
| 8 | Rahmen |
| 9 | Wandung |
| 9' | Handhabe (von 6' bzw. 11) |
| 10 | Kappe |

| 11 | Ventil |
|---|---|
| 11' | Kammer |
| 12 | Gasflasche |
| 12' | Kammer |
| 13 | Kammer |
| 13' | Ventilscheibenstütze |
| 14 | Außenseite (von 3) |
| 14' | Folienabschnitt |
| 15 | — |
| 16 | Hautabschnitt |
| 17 | Patient |
| 18 | Außenseite (von 3) |
| 19 | Widerlager |
| 20 | — |
| 21 | Polsterung (auf 14) |
| 22 | Seitenfläche |
| 23 | Zahnlücke |
| 24 | Stirnplatten |
| 25 | Strömungsmittel-Zuführleitung |
| 25' | Abschnitt (mit 4'; von 25) |
| 26 | Flansch |
| 27 | Durchgangsöffnung (in 25; für 9') |
| 28 | Verschlußkörper (in 25) |
| 29 | Umhüllung (von 1) |
| 30 | Schweißnähte (von 29) |

**Patentansprüche**

1. Druckverband zum Stillen einer blutenden äußeren Wunde, mit einer hohlen, gasdichten Kompresse (2), die wenigstens einen mit seiner Außenseite auf die zu versorgende Wunde (4) aufzulegenden, elastisch verformbaren Wandungsabschnitt (3) aufweist, der durch innenseitige Beaufschlagung mit einem unter Überdruck stehenden Strömungsmittel nach außen zu verformen ist, wobei die Kompresse (2) zur Erzeugung des inneren Überdruckes mit einem Ventil (11) versehen ist, dadurch gekennzeichnet, daß der Ventilkörper des Ventils (11) aus einer flexiblen Ventilscheibe (6) besteht, die an der an den Strömungsquerschnitt (2') angrenzenden Wandung einer Strömungsmittel-Zuführleitung (25) so befestigt ist, daß sie bei einem inneren Überdruck der Kompresse (2) dichtend an die an den Strömungsquerschnitt (2') angrenzende, einen Ventilsitz (4') bildende Wandung anzudrücken ist, und daß sie bei einem äußeren Überdruck an wenigstens einem Abschnitt des Ventilsitzes (4') außer Anlage mit dem Ventilsitz (4') zu bringen ist.

2. Druckverband nach Anspruch 1, dadurch gekennzeichnet, daß die Ventilscheibe (6') aus einem (gummi-)elastischen Material besteht.

3. Druckverband nach Anspruch 1 oder 2, dadurch gekennzeichnet, daß der Ventilsitz (4') (gummi-)elastisch ausgebildet ist.

4. Druckverband nach einem der Ansprüche 1 bis 3, dadurch gekennzeichnet, daß der Ventilsitz (4') ringförmig ausgebildet ist.

5. Druckverband nach einem der Ansprüche 1 bis 4, dadurch gekennzeichnet, daß der Ventilsitz (4') die der Ventilscheibe (6') zugekehrte Stirnseite eines Abschnittes (25') der Strömungsmittel-Zuführleitung (25) ist.

6. Druckverband nach einem der Ansprüche 1 bis 4, dadurch gekennzeichnet, daß der Ventilsitz (4') ein der Ventilscheibe (6') zugekehrter Flansch der Strömungsmittel-Zuführleitung (25) ist.

7. Druckverband nach einem der Ansprüche 1 bis 6, dadurch gekennzeichnet, daß die Ventilscheibe (6') an einem Umfangsabschnitt (6'') ihres äußeren Randabschnittes mit der Strömungsmittel-Zuführleitung (25) gasdicht verbunden ist.

8. Druckverband nach Anspruch 7, dadurch gekennzeichnet, daß die Ventilscheibe (6') an mehreren Umfangsabschnitten (6'') ihres äußeren Randabschnittes mit der Strömungsmittel-Zuführleitung (25) gasdicht verbunden ist.

9. Druckverband nach Anspruch 7 oder 8, dadurch gekennzeichnet, daß der nicht mit der Strömungsmittel-Zuführleitung (25) verbundene Umfangsabschnitt (6''') der Ventilscheibe (6') kleiner ist als der mit der Strömungsmittel-Zuführleitung (25) gasdicht verbundene Umfangsabschnitt (6'').

10. Druckverband nach einem der Ansprüche 1 bis 9, dadurch gekennzeichnet, daß die Ventilscheibe (6') im Bereich eines nicht mit der Strömungsmittel-Zuführleitung (25) verbundenen Randabschnittes (6''') mit einer Handhabe (9') versehen ist, mittels welcher der nicht mit der Strömungsmittel-Zuführleitung (25) verbundene Randabschnitt (6''') — auch bei innerem Überdruck der Kompresse (2) — außer Anlage mit dem Ventilsitz (4) zu bringen ist.

11. Druckverband nach Anspruch 10, dadurch gekennzeichnet, daß die Handhabe (9') integraler Bestandteil der Ventilscheibe (6') ist.

12. Druckverband nach Anspruch 11, dadurch gekennzeichnet, daß die Handhabe (9') als nach außen über die Strömungsmittel-Zuführleitung (25) vorstehender Ansatz ausgebildet ist.

13. Druckverband nach Anspruch 12, dadurch gekennzeichnet, daß die Handhabe (9') sich durch eine Durchgangsöffnung der Strömungsmittel-Zuführleitung (25) erstreckt.

14. Druckverband nach Anspruch 13, dadurch gekennzeichnet, daß die Durchgangsöffnung (27) mittels eines gummi-elastischen Folienabschnittes (14') nach außen gasdicht abgedichtet ist, die an ihrem Rand umlaufend mit der Außenseite der Strömungsmittel-Zuführleitung (25) gasdicht verbunden ist.

15. Druckverband nach einem der Ansprüche 1 bis 14, dadurch gekennzeichnet, daß auf der der Ventilscheibe (6') abgekehrten Seite des Ventilsitzes (4') eine Ventilscheibenstütze (13') vorgesehen ist.

16. Druckverband nach Anspruch 15, dadurch gekennzeichnet, daß die Ventilscheibenstütze (13') aus wenigstens einem Ansatz der Strömungsmittel-Zuführleitung (25) besteht.

17. Druckverband nach Anspruch 15 oder 16, dadurch gekennzeichnet, daß die Ventilscheibenstütze (13') aus wenigstens einer parallel zur Ebene des Ventilsitzes (4') verlaufenden Traverse besteht.

18. Druckverband nach Anspruch 17, dadurch gekennzeichnet, daß die Ventilscheibenstütze (13') aus wenigstens zwei im Winkel zueinander verlaufenden Traversen besteht.

19. Druckverband nach Anspruch 18, dadurch gekennzeichnet, daß zwei im wesentlichen rechtwinklig zueinander verlaufende Traversen eine Ventilscheibenstütze (13') bilden.

20. Druckverband nach Anspruch 15, dadurch gekennzeichnet, daß die Ventilscheibenstütze (13') aus einem im wesentlichen parallel zur Ebene des Ventilsitzes (4') verlaufenden Maschengewebe besteht.

21. Druckverband nach einem der Ansprüche 1 bis 20, dadurch gekennzeichnet, daß das Ventil (11) an eine Gasflasche (12) anzuschließen ist.

22. Druckverband nach einem der Ansprüche 1 bis 21, dadurch gekennzeichnet, daß am Ende der Strömungsmittel-Zuführleitung (25) ein die Strömungsmittel-Zuführleitung (25) gasdicht verschließender Verschlußkörper (28) anzuordnen ist.

23. Druckverband nach einem der Ansprüche 1 bis 22, dadurch gekennzeichnet, daß die Kompresse (2) an wenigstens zwei Wandungsabschnitten elastisch verformbar ausgebildet ist.

24. Druckverband nach Anspruch 23, dadurch gekennzeichnet, daß die beiden elastisch verformbaren Wandungsabschnitte (3, 9) nicht in einer Ebene liegen.

25. Druckverband nach Anspruch 24, dadurch gekennzeichnet, daß die beiden elastisch verformbaren Wandungsabschnitte (3, 9) im wesentlichen parallel zueinander verlaufen.

26. Druckverband nach Anspruch 24 oder 25, dadurch gekennzeichnet, daß die elastisch verformbaren Wandungsabschnitte (3, 9) im Winkel zueinander verlaufen.

27. Druckverband nach einem der Ansprüche 1 bis 26, dadurch gekennzeichnet, daß die Kompresse (2) insgesamt aus einem elastisch verformbaren Material besteht.

28. Druckverband nach Anspruch 25 oder 26, dadurch gekennzeichnet, daß die Kompresse (2) balgartig ausgebildet ist und ihrem im wesentlichen rechtwinklig zur Balgachse verlaufenden, mit seiner Außenseite auf die zu versorgende Wunde aufzulegenden Wandungsabschnitt (21, 24) formfest ausgebildet ist.

29. Druckverband nach Anspruch 28, dadurch gekennzeichnet, daß ein formfester Kompressenabschnitt (24) an seiner der zu versorgenden Wunde (4) zugekehrten Außenseite mit einer Polsterung (21) versehen ist.

30. Druckverband nach einem der Ansprüche 1 bis 29, gekennzeichnet durch einen den (die) verformbaren Wandungsabschnitt(e) (3) gasdicht haltenden Rahmen (8).

31. Druckverband nach einem der Ansprüche 1 bis 30, dadurch gekennzeichnet, daß das Ventil (11) an eine Gasflasche (12) anzuschließen ist.

32. Druckverband nach einem der Ansprüche 1 bis 31, dadurch gekennzeichnet, daß am Ende der Strömungsmittel-Zuführleitung (25) ein die Strömungsmittel-Zuführleitung (25) gasdicht verschließender Verschlußkörper (28) anzuordnen ist.

33. Druckverband nach einem der Ansprüche 1 bis 32, dadurch gekennzeichnet, daß der einer zu versorgenden Wunde (4) zugekehrte Wandungsabschnitt der Kompresse (2) im wesentlichen ringförmig ausgebildet ist.

34. Druckverband nach einem der Ansprüche 1 bis 33, gekennzeichnet durch eine sterile Umhüllung.

35. Druckverband nach einem der Ansprüche 1 bis 34, gekennzeichnet durch eine Bevorratung in einem sterilen Verbandspäckchen.

## Claims

1. A pressure dressing for staunching a bleeding external wound and comprising a hollow gas-tight compress (2) having at least one resiliently deformable wall portion (3), the outer side of which is to be laid on the wound (4) to be dressed, and the dressing being externally deformable by being internally acted upon by a flow medium under excess pressure, the compress (2) comprising a valve (11) for generating the internal excess pressure, characterised in that the body of the valve (11) comprises a flexible valve disc (6') which is secured to the walls of a flow-medium supply pipe (25) adjacent the flow cross-section (2') so that when there is an excess internal pressure on the compress (2), the valve disc is pressed in sealing-tight manner against the wall adjacent the flow cross-section (2') and forming a valve seat (4'), whereas when there is an external excess pressure on at least one portion of the valve seat (4'), the valve disc is brought out of engagement therewith.

2. A pressure dressing according to claim 1, characterised in that the valve disc (6') is made of resilient or elastomeric material.

3. A pressure dressing according to claim 1 or 2, characterised in that the valve seat (4') is resilient or elastomeric.

4. A pressure dressing according to any of claims 1 to 3, characterised in that the valve seat (4') is annular.

5. A pressure dressing according to any of claims 1 to 4, characterised in that the valve seat (4') is in the end face of a portion (25') of the flow-medium supply pipe (25) facing the valve disc (6').

6. A pressure dressing according to any of claims 1 to 4, characterised in that the valve seat (4') is a flange of the flow-medium supply pipe (25) facing the valve disc (6').

7. A pressure dressing according to any of claims 1 to 6, characterised in that a peripheral portion (6'') of the outer edge portion of the valve disc (6') is connected in gas-tight manner to the flow-medium supply pipe (25).

8. A pressure dressing according to claim 7, characterised in that a number of peripheral portions (6'') of the outer edge portion of the valve disc (6') are connected in gas-tight manner to the flow-medium supply pipe (25).

9. A pressure dressing according to claim 7 or 8, characterised in that the peripheral portion (6''') of the valve disc (6') not connected to the flow-medium supply pipe (25) is smaller than the peripheral portion (6'') connected in gas-tight manner to the flow-medium supply pipe (25).

10. A pressure dressing according to any of claims 1 to 9, characterised in that an edge portion (6''') of the valve disc (6') not connected to the flow-medium supply pipe (25) is provided with a handle (9') by means of which the edge portion (6''') not connected to the flow-medium supply pipe (25) can be brought out of engagement with the valve seat (4') even when there is internal excess pressure on the compress (2).

11. A pressure dressing according to claim 10, characterised in that the handle (9') is an integral component of the valve disc (6').

12. A pressure dressing according to claim 11, characterised in that the handle (9') is an attachment externally projecting beyond the flow-medium supply pipe (25).

13. A pressure dressing according to claim 12, characterised in that the handle (9') extends through an opening in the flow-medium supply pipe (25).

14. A pressure dressing according to claim 13, characterised in that the opening (27) is sealed in gas-tight manner from the exterior by an elastomeric foil portion (14'), which is peripherally connected in gas-tight manner at its edge to the outside of the flow-medium supply pipe (25).

15. A pressure dressing according to any of claims 1 to 14, characterised in that a valve-disc support (13') is provided on the side of the valve seat (4') remote from the valve disc (6').

16. A pressure dressing according to claim 15, characterised in that the valve-disc support (13') comprises at least one attachment on the flow-medium supply pipe (25).

17. A pressure dressing according to claim 15 or 16, characterised in that the valve-disc support (13') comprises at least one cross-member extending parallel to the plane of the valve seat (4').

18. A pressure dressing according to claim 17, characterised in that the valve-seat support (13') comprises at least two cross-members extending at an angle to one another.

19. A pressure dressing according to claim 18, characterised in that two cross-members extending substantially at right angles to one another constitute a valve-seat support (13').

20. A pressure dressing according to claim 15, characterised in that the valve-seat support (13') comprises a mesh extending substantially parallel to the plane of the valve seat (4').

21. A pressure dressing according to any of claims 1 to 20, characterised in that the valve (11) is for connecting to a gas cylinder (12).

22. A pressure dressing according to any of claims 1 to 21, characterised in that a closure member (28) for closing the flow-medium supply pipe (25) in gas-tight manner is provided at the end thereof.

23. A pressure dressing according to any of claims 1 to 22, characterised in that at least two wall portions of the compress (2) are made resiliently deformable.

24. A pressure dressing according to claim 23, characterised in that the two resiliently deformable wall portions (3, 9) do not lie in the same plane.

25. A pressure dressing according to claim 24, characterised in that the two resiliently deformable wall portions (3, 9) are substantially parallel to one another.

26. A pressure dressing according to claim 24 or 25, characterised in that the resiliently deformable wall portions (3, 9) extend at an angle to one another.

27. A pressure dressing according to any of claims 1 to 26, characterised in that the compress (2) as a whole consists of resiliently deformable material.

28. A pressure dressing according to claim 25 or 26, characterised in that the compress (2) is bellows-like and is given a non-deformable wall portion (21, 24) extending substantially at right angles to the bellows axis and adapted to be laid with its outside on the wound to be dressed.

29. A pressure dressing according to claim 28, characterised in that a non-deformable compress portion (24) is padded (21) on its outer side facing the wound (4) to be dressed.

30. A pressure dressing according to any of claims 1 to 29, characterised by a frame (8) which holds the deformable frame portion or portions (3) in gas-tight manner.

31. A pressure dressing according to any of claims 1 to 30, characterised in that the valve (11) is for connecting to a gas cylinder (12).

32. A pressure dressing according to any of claims 1 to 31, characterised in that a closure member (28) for closing the flow-medium supply pipe (25) in gas-tight manner is provided at the end thereof.

33. A pressure dressing according to any of claims 1 to 32, characterised in that the wall portion of the compress (2) facing a wound (4) to be dressed is made substantially circular.

34. A pressure dressing according to any of claims 1 to 33, characterised by a sterile wrapper.

35. A pressure dressing according to any of claims 1 to 34, characterised by storage in a sterile first-aid packet.

## Revendications

1. Garrot pour faire cesser l'écoulement de sang d'une blessure externe, comprenant une compresse (2) creuse, étanche aux gas, qui présente au moins une section de paroi (3) déformable élastiquement, à appliquer sur la blessure (4) à soigner par sa face externe, et qui est à déformer vers l'extérieur par attaque de l'intérieur avec un agent d'écoulement mis sous pression, la compresse (2) étant munie, pour produire la surpression interne, d'une soupape (11), caractérisé par le fait que le corps de la soupape (11) est constitué par un disque (6'), qui est fixé à la paroi aboutissant à la section d'écoulement (2'), d'un conduit d'amenée (25) d'agent d'écoulement de telle sorte que, lors d'une surpression interne, la compresse (2) s'applique de manière étanche contre la paroi formant siège de soupape (4') adjacente à la section d'écoulement (2') et que, par une surpression externe, elle soit mise hors contact du siège de soupape (4') sur au moins une section de ce siège (4').

2. Garrot selon la revendication 1, caractérisé par le fait que le disque de soupape (6') est en un matériau élastique (caoutchouteux).

3. Garrot selon la revendication 1 ou 2, caractérisé par le fait que le siège de soupape (4') est à structure élastique (caoutchouteux).

4. Garrot selon l'une des revendications 1 à 3, caractérisé par le fait que le siège de soupape (4') est de forme annulaire.

5. Garrot selon l'une des revendications 1 à 4, caractérisé par le fait que le siège de soupape (4') est la face frontale, tournée vers le disque de soupape (6'), d'une section (25') du conduit d'amenée (25) de l'agent d'écoulement.

6. Garrot selon l'une des revendications 1 à 4, caractérisé par le fait que le siège de soupape (4') est une bride, tournée vers le disque de soupape (6'), du conduit d'amenée (25) d'agent d'écoulement.

7. Garrot selon l'une des revendications 1 à 6, caractérisé par le fait que le disque de soupape (6') est relié, de façon étanche aux gaz, au conduit d'amenée (25), d'agent d'écoulement par une section périphérique (6'') de sa section de bord externe.

8. Garrot selon la revendication 7, caractérisé par le fait que le disque de soupape (6') est relié, de façon étanche aux gaz, au conduit d'amenée (25), d'agent d'écoulement par plusieurs sections périphériques (6'') de sa section de bord externe.

9. Garrot selon les revendications 7 ou 8, caractérisé par le fait que la section périphérique (6''') du disque de soupape (6'), qui n'est pas reliée au conduit (25) d'amenée d'agent d'écoulement, est plus petite que la section périphérique reliée, de façon étanche aux gaz, au conduit d'amenée (25) d'agent d'écoulement.

10. Garrot selon l'une des revendications 1 à 9, caractérisé par le fait que le disque de soupape (6') est muni, au voisinage d'une section de bord (6''') non reliée au conduit d'amenée (25) d'agent d'écoulement, d'une prise (9') grâce à laquelle la section de bord (6''') non reliée au conduit d'amenée (25) d'agent d'écoulement — même en cas de suppression de la compresse (2) — est mise hors contact du siège de soupape (4').

11. Garrot selon la revendication 10, caractérisé par le fait que la prise (9') est une partie constitutive intégrale du disque de soupape (6').

12. Garrot selon la revendication 11, caractérisé par le fait que la prise (9') est réalisée sous forme d'une appendice, en saillie vers l'extérieur, sur le conduit d'amenée (25) d'agent d'écoulement.

13. Garrot selon la revendication 12, caractérisé par le fait que la prise (9') s'étend à travers un orifice de passage du conduit d'amenée (25) d'agent d'écoulement.

14. Garrot selon la revendication 13, caractérisé par le fait que l'orifice de passage (27) est rendu étanche aux gaz vers l'extérieur au moyen d'une portion de feuille (14') élastique (caoutchouteux), qui est reliée, de façon étanche aux gaz, avec la face externe du conduit d'amenée (25) de l'agent d'écoulement, qu'elle suit par son bord.

15. Garrot selon l'une des revendications 1 à 14, caractérisé par le fait que la face du siège de soupape (4'), écartée du disque de soupape (6'), est munie d'un support de disque (13').

16. Garrot selon la revendication 15, caractérisé par le fait que le support (13') du disque de soupape est constitué par au moins un appendice du conduit d'amenée (25) d'agent d'écoulement.

17. Garrot selon la revendication 15 ou 16, caractérisé par le fait que le support (13') de disque de soupape est constitué par au moins une traverse parallèle au plan du siège de soupape (4').

18. Garrot selon la revendication 17, caractérisé par le fait que le support (13') de disque de soupape est constitué par au moins deux traverses faisant un angle entre elles.

19. Garrot selon la revendication 18, caractérisé par le fait que deux traverses, s'étendant à peu près à angle droit, l'une par rapport à l'autre, forment un support (13') de disque de soupape.

20. Garrot selon la revendication 13, caractérisé par le fait que le support (13') de disque de soupape est constitué par un treillis s'étendant à peu près parallèlement au plan du siège de soupape (4').

21. Garrot selon l'une des revendications 1 à 20, caractérisé par le fait que la soupape (11) est à raccorder à une bouteille de gaz (12).

22. Garrot selon l'une des revendications 1 à 21, caractérisé par le fait qu'à l'extrémité du conduit (25) d'agent d'écoulement est à monter un corps d'obturation (28) obturant, de façon étanche aux gaz, le conduit (25) d'amenée d'agent d'écoulement.

23. Garrot selon l'une des revendications 1 à 22, caractérisé par le fait que la compresse (2) est conçue déformable élastiquement par au moins deux de ses portions de paroi.

24. Garrot selon la revendication 23, caractérisé par le fait que les deux portions de paroi déformables élastiquement (3, 9) ne sont pas dans un même plan.

25. Garrot selon la revendication 24, caractérisé par le fait que les deux portions de paroi déformables élastiquement (3, 9) sont sensiblement parallèles entre elles.

26. Garrot selon la revendication 24 ou 25, caractérisé par le fait que les deux portions de paroi (3, 9) déformables élastiquement forment entre elles un angle.

27. Garrot selon l'une des revendications 1 à 26, caractérisé par le fait que la compresse (2) est globalement en un matériau déformable élastiquement.

28. Garrot selon la revendication 25 ou 26, caractérisé par le fait que la compresse (2) est en forme de soufflet et sa portion de paroi (21, 24) à appliquer sur la blessure à soigner par sa face externe, et s'étendant sensiblement à angle droit par rapport à l'axe du soufflet, est réalisée de forme rigide.

29. Garrot selon la revendication 28, caractérisé par le fait qu'une portion de compresse (24) de forme rigide est munie d'un rembourrage (21) sur sa face externe tournée vers la blessure à soigner (4).

30. Garrot selon l'une des revendications 1 à 29, caractérisé par un cadre (8) supportant, de façon étanche aux gaz, la (les) portion(s) de paroi déformable(s) (3).

31. Garrot selon l'une des revendications 1 à 30, caractérisé par le fait que la soupape (11) est à raccorder à une bouteille à gaz (12).

32. Garrot selon l'une des revendications 1 à 31, caractérisé par le fait qu'à l'extrémité du conduit (25) d'agent d'écoulement est à monter un corps d'obturation (28) obturant de façon étanche aux gaz le conduit (25) d'amenée d'agent d'écoulement.

33. Garrot selon l'une des revendications 1 à 32, caractérisé par le fait que la portion de la compresse (2) tournée vers la blessure à soigner est sensiblement de forme circulaire.

34. Garrot selon l'une des revendications 1 à 33 caractérisé par une enveloppe stérile.

35. Garrot selon l'une des revendications 1 à 34 caractérisé par un approvisionnement dans un paquet de pansement.

FIG.1

FIG.2

FIG.3

FIG.4

FIG.5

FIG.6

FIG.7

FIG.8

FIG.9

FIG.10

FIG.11